# EUROPEAN PATENT APPLICATION

(11) **EP 2 438 854 A2**
(43) Date of publication of application: **11.04.2012**
(21) Application number: 11184353.8
(22) Date of filing: 07.10.2011
(51) Int. Cl.: A61B 5/0432, A61B 5/0404, A61B 5/0408, A61B 5/0428

(54) **Ambulatory electrocardiographic monitor and method of use**

(30) Priority: 08.10.2010 US 901444
(71) Applicant: Cardiac Science Corporation, Bothell, WA 98021 (US)
(72) Inventor: Bishay, Jon Mikalson, Seattle, WA Washington 98119 (US); Bardy, Gust H., Carnation, WA Washington 98014 (US); Felix, Jason, Vashon Island, WA Washington 98070 (US)
(74) Representative: Curley, Donnacha John

(57) **Abstract**

A method for performing ambulatory electrocardiographic monitoring is provided. An ambulatory ECG monitor (11), that includes a plurality of sensing electrodes (48) coupled to self-powered sensing circuitry (71), is provisioned. A monitoring site (15) is located on the surface of a patient's chest at midline and above the body of the sternum adjacent to the fourth and fifth intercostal spaces. The electrodes (48) are aligned and placed along the midline. The ambulatory ECG monitor (11) is removably adhered to the monitoring site (15) for the duration of monitoring. ECG data is sensed through the sensing electrodes (48) at the monitoring site (15) and the sensed ECG data is recorded into the sensing circuitry (71).

## Description

### Field

This application relates in general to ambulatory electrocardiography and, in particular, to an ambulatory electrocardiographic monitor and method of use.

### Background

The cardiac electrical signal begins in the cells of the sinoatrial node in the right atrium. These cells spontaneously depolarize and create a cardiac action potential of electrical impulses that rapidly propagates outward across the right atrium and then the left atrium. The cardiac action potential in turn stimulates muscle cells of the atrial myocardium to depolarize and contract to push blood into the ventricles. Shortly thereafter, this atrial action potential encounters the atrioventricular node located at the juncture of the atria and ventricles near the center of the heart. The atrioventricular node slightly delays cardiac action potential propagation into the ventricles to ensure complete drainage of blood from the atria. Thereafter, the muscle cells of the ventricular myocardium are activated by the electrical wave front and are stimulated into systolic contraction. After a rest and reset period, the complete the heart beat cycle repeats. Any disruption in this process, which can include heart block, sinus bradycardia, atrial fibrillation, and ventricular tachycardia, can lead to the symptoms ranging from dizziness to a sensation of heart fluttering or palpitations, loss of consciousness or even death. Being able to record the electrical signal of the heart is a fundamental diagnostic tool of every physician.

Identifying abnormal rhythms depends upon the manner in which and the amplitude of the depolarization signal of the muscle cells of the atrial and ventricular myocardium that in turn act as sequential voltage sources, which generate a current flow across the thoracic region of the body and result in a characteristic signal on the body surface. In a typical electrocardiographic (ECG) monitor, cardiac action potentials occur between 0.05 Hz to 150Hz with a signal strength of around 3mVp-p (peak-to-peak). Although miniscule, the current flow can be measured to characterize the electrical activity of the heart using an ECG monitor or similar device. Voltage differentials from pairings of the electrodes are filtered, amplified, and combined into P, QRS, and T complexes.

Conventionally, cardiac action potentials are detected through electrodes attached to the skin on the chest and limbs based on the American Heart Association's classic 12-lead placement model, such as P. Libby et al., "Braunwald's Heart Disease - A Textbook of Cardiovascular Medicine," Chs. 11 and 12 (8th ed. 2008), the disclosure of which is incorporated by reference. Both traditional in-clinic and ambulatory Holter-style ECG monitors follow the standard 12-lead model with variations on numbers and placement of leads. Generally, limb lead electrodes are placed on each arm and on the left leg, while precordial lead electrodes are placed on the left upper chest region over the heart in close proximity to the heart and at a location of strongest ventricular cardiac action potential signal strength. In turn, the monitoring circuitry relies on the superior signal strength from over-the-heart electrode placement and the relatively long signal vector length that is afforded by lead placement over a wider physical expanse of the body. For instance, based upon the large inter-electrode distances, signal amplification assumes a signal strength of around 3mVp-p (peak-to-peak).

The 12-lead placement model, however, is poorly suited to long-term ambulatory monitoring both from the perspective of comfort and from the perspective of reliability. The latter concern simply relates to how standard monitoring electrodes fall off with modest movement. In-clinic ECG monitoring, for instance, assumes that the patient will remain relatively stationary and that the limb leads can be repositioned as necessary to provide sufficient electrode separation for recording a signal of reasonable amplitude. In contrast, during ambulatory monitoring, a patient's body is in continual motion, even during sleep, albeit to a lesser degree. Electrodes are apt to detach and signal quality degrades or is absent altogether.

Holter and other forms of ambulatory ECG monitors generally rely on electrodes placed close to the heart as suggested by the 12-lead placement model. For instance, U.S. Patent No. 3,215,136 issued November 2, 1965 to Holter et al. discloses an electrocardiographic recording and playback means. Episodes of ventricular tachycardia, asystolic intervals, and ectopic heart activities are sensed by electrodes disposed on the patient's skin in a suitable location, with sufficient inter-electrode separation. These signals are ordinarily recorded via a compact recorder worn by the patient that records an electrocardiogram (ECG) while he engages in activities of daily living, which subsequently allows a cardiac specialist to temporally correlate patient symptoms and cardiac abnormalities with activities. A cardiac rhythm disorder, as well as the absence of a rhythm disorder during symptoms, can sometimes be identified by having the patient record those symptoms during the use of the Holter monitor.

U.S. Patent No. 6,117,077 issued September 12, 2000 to Del Mar et al. discloses a long-term ambulatory physiological recorder provided in a relatively planar and triangular-shaped recorder housing with three adhesive electrode pads. The recorder is fully self-contained and mounted immediately adjacent to the organ system that is to be monitored. Electrode pads are adhesively and conductively attached to the patient's left chest in a position generally over the heart with positive and negative terminals in a relative vertical position from the top to the bottom of the heart. Additional electrode leads can also be connected to an input port on the recorder and placed over adjacent areas of the upper chest.

U.S. Patent No. 6,456,872 issued September 24, 2002 to Faisandier discloses a Holter-type apparatus for recording physiological signals indicative of cardiac activity. A base unit is formed of a flexible sheet carrying electrodes and a recording case that carries a battery and flexible printed circuit material. The base unit is disposable and can be changed with each new patient examination. The recorder case is fixed in position on the patient's thorax through a plurality of electrodes affixed either through adhesion or through depression using suction cups. Alternatively, the base unit can be carried by a thoracic belt or a hanging strap collar. The recording case includes electronic circuits for the collection and processing of ECG signals and a data transmission port is provided for bi-directional exchange of data, control parameters, and information.

U.S. Patent No. 7,257,438 issued August 14, 2007 to Kinast discloses a patient-worn medical monitoring device that includes a lanyard and electronics package supported in the manner of a pendant. A lanyard includes integral electrodes or other sensors for making physiological measurements, which may be stored in a monitor for later readout or transmitted, before or after processing, to a remote location. The device can locally process and analyze a patient's signals and transmit only summary data or analyzed results to a remote device.

U.S. Patent application, Publication No. 2007/0255153, filed November 1, 2007, to Kumar et al.; U.S. Patent application, Publication No. 2007/0225611, filed February 6, 2007, to Kumar et al.; and U.S. Patent application, Publication No. 2007/0249946, filed February 6, 2007, to Kumar et al. disclose a non-invasive cardiac monitor and methods of using continuously recorded cardiac data. A heart monitor suitable for use in primary care includes a self-contained and sealed housing. The housing encloses an electronic memory connected to electrodes on the upper left chest to detect an ECG. A thin, flexible, and tapered rim or lip is provided around the edges of the electronics portion of the monitor to increase the surface area available for adhesion. Continuously recorded cardiac monitoring is provided through a sequence of simple detect-store-offload operations that are performed by a state machine. The housing is adapted to remain affixed to a patient for at least seven days. The heart monitor can include an activation or event notation button, the actuation of which increases the fidelity of the ECG information stored in the memory. The stored information can be retrieved and analyzed offline to identify both normal and abnormal ECG events. The monitor is specifically intended to provide monitoring continuously and without interruption over an extended period. Despite the improvement in size and ease of use of such a system, neither this device or any of the above described systems defines a device capable of extremely simple and reliable application for any body habitus and by any individual regardless of training.

Finally, U.S. Patent application, Publication No. 2008/0284599, filed April 28, 2006, to Zdeblick et al. and U.S. Patent application, Publication No. 2008/0306359, filed December 11, 2008, to Zdeblick et al., disclose a pharma-informatics system for detecting the actual physical delivery of a pharmaceutical agent into a body. An integrated circuit is surrounded by pharmacologically active or inert materials to form a pill, which dissolve in the stomach through a combination of mechanical action and stomach fluids. As the pill dissolves, areas of the integrated circuit become exposed and power is supplied to the circuit, which begins to operate and transmit a signal that may indicate the type, A signal detection receiver can be positioned as an external device worn outside the body with one or more electrodes attached to the skin at different locations. The receiver can include the capability to provide both pharmaceutical ingestion reporting and psychological sensing in a form that can be transmitted to a remote location, such as a clinician or central monitoring agency.

Therefore, a need remains for an ambulatory ECG monitoring device and method of use adapted to long term monitoring that resists body movement while providing ease and discreteness of use and patient comfort regardless of patient knowledge and regardless of patient body habitus.

### Summary

A small and anatomically adaptive ambulatory ECG monitor is applied in-clinic by a primary care provider, by the patient at home, or by other healthcare or lay individuals to record ECG data over an extended time period, while the patient engages in activities of daily living. The ECG monitor is placed on the patient's chest at midline, covering the center third of the sternum and centered between the manubrium and the xiphoid process on the inferior border of the sternum. This unique location for ECG monitor application and the monitor's small size allow for a uniformity of applicability by minimally trained physicians or even lay individuals. Upon completion of monitoring, the patient delivers the monitor to a monitoring, consultation, and specialist referral center ("referral center"), along with encoded patient medical information and a diary recording the patient's subjective impressions contemporaneous to the monitoring, such as described in commonly-assigned U.S. Patent application, entitled "Computer-Implemented System And Method For Evaluating Ambulatory Electrocardiographic Monitoring of Cardiac Rhythm Disorders," Serial No. 12/901,461, filed October 8, 2010, pending, the disclosure of which is incorporated by reference. A unique identifier is assigned to the monitor that is used throughout the remainder of the diagnosis and referral process. The referral center interprets the ECG data and patient medical information and, where indications of a cardiac rhythm disorder or other health concern arise, an automated referral to a cardiac specialist, or other healthcare specialist, is made. The patient can proactively track the status of and make inquiries concerning his test results through the unique identifier. The primary care physician is also informed of the referral.

One embodiment provides a method for performing ambulatory ECG monitoring. An ambulatory ECG monitor, that includes a plurality of sensing electrodes coupled to self-powered sensing circuitry, is provisioned. A monitoring site is located on the surface of a patient's chest at the sternal midline adjacent to the fourth and fifth intercostal spaces. The electrodes are aligned and placed along the midline. The ambulatory ECG monitor is removably adhered to the skin for the duration of monitoring. ECG data is sensed through the sensing electrodes at the monitoring site and the sensed ECG data is recorded into the sensing circuitry.

A further embodiment provides a method for performing ambulatory ECG monitoring at a midline sternum-centered location. An ambulatory ECG monitor, that includes a plurality of sensing electrodes coupled to self-powered sensing circuitry and enclosed in a flexible housing, is provisioned. The flexibility of the housing is integral to the design to comfortably adhere to the sternal surface. The sternal surface is non-planar, even in men, and the surface of the skin over the sternum has a subtle three-dimensional topography. A proper understanding of this topography is critical to device design, as provided through the flexible housing, to ensure that ECGs can be recorded from the sternal location. A layer of skin adhesive is independently suspended from a bottom of the flexible housing. A monitoring site is located on the surface of a patient's chest at midline and adjacent to the fourth and fifth intercostal spaces, a location ideal for recording both atrial and ventricular cardiac signals. The ambulatory ECG monitor is conformably placed in this location. The electrodes are aligned and placed along the midline. The skin adhesive is removably adhered to the monitoring site for the duration of monitoring. The housing is axially and laterally bendable along the non-planar contours of the monitoring site. ECG data is sensed through the sensing electrodes at the monitoring site and the sensed ECG data is recorded into the sensing circuitry.

A still further embodiment provides another form of an ambulatory ECG monitor. Self-powered ECG sensing circuitry is provided. A plurality of sensing electrodes is coupled to the sensing circuitry. A housing encloses the sensing circuitry. A skin adhesive layer faces a contact surface and is independently suspended from the housing.

A yet even further embodiment provides an ambulatory ECG monitor with conformal shape and independent suspension. A flexible ECG circuitry body includes self-powered ECG sensing circuitry including a processor, memory, and finite power supply, a circuit board that exhibits axial and lateral flexibility necessary for sternal application and upon which the sensing circuitry is included, and a housing that encloses the circuit board. A plurality of sensing electrodes is coupled to the processor, which processes and stores sensed ECG data into the memory. A skin adhesion assembly includes a layer of skin adhesive facing a contact surface and a set of standoffs affixed to and defining a gap between the skin adhesive layer and a bottom surface of the housing of the circuitry body.

An ambulatory ECG monitor in accordance with foregoing embodiments can be built at low cost, size and weight with a bill of materials of about one fifth of the cost of a conventional ambulatory ECG monitor. Low cost enables clinics and hospitals to maintain amble inventory at all times, thereby facilitating the ebb and flow of patients in need of ambulatory ECG monitoring who will not need to wait on monitor availability or laboratory staffing for use and overread.

Additionally a single-use ECG monitor in the form of an adhesive patch in accordance with foregoing embodiments can be constructed with a weight of less than two ounces and inter-electrode spacing of less than 6cm, which presents three advantages. First, costs for shipping the monitors to clinics, hospitals, pharmacies, and other locations are reduced, especially when large quantities must be mailed around the world. Second, small size and weight ambulatory ECG monitors can be easily carried in the pockets of health care providers and therefore applied upon demand without the need to either retrieve the monitors from a special location or to send the patient to a separate laboratory. Third, small, lightweight ambulatory ECG monitors reduce shear forces on the skin, which further ensures good signal acquisition and long-term ECG recording by facilitating adherence to the skin and comfort for the patient.

Still other embodiments will become readily apparent to those skilled in the art from the following detailed description, wherein are described embodiments by way of illustrating the best mode contemplated. As will be realized, other and different embodiments are possible and the embodiments' several details are capable of modifications in various obvious respects, all without departing from their spirit and the scope. Accordingly, the drawings and detailed description are to be regarded as illustrative in nature and not as restrictive.

### Brief Description of the Drawings

FIGURE 1 is a front anatomical diagram showing placement of an ambulatory electrocardiographic monitor on a male patient.
FIGURE 2 is a cutaway anatomical diagram showing placement of the ambulatory electrocardiographic monitor of FIGURE 1.
FIGURE 3 is an exploded perspective view of an ambulatory electrocardiographic monitor in accordance with one embodiment.
FIGURE 4 is a side view of the ambulatory electrocardiographic monitor of FIGURE 3.
FIGURE 5 is a bottom view of the ambulatory electrocardiographic monitor of FIGURE 3.
FIGURE 6 is a functional block diagram showing the groups of electronic component of the ambulatory electrocardiographic monitor of FIGURE 3.

### Detailed Description

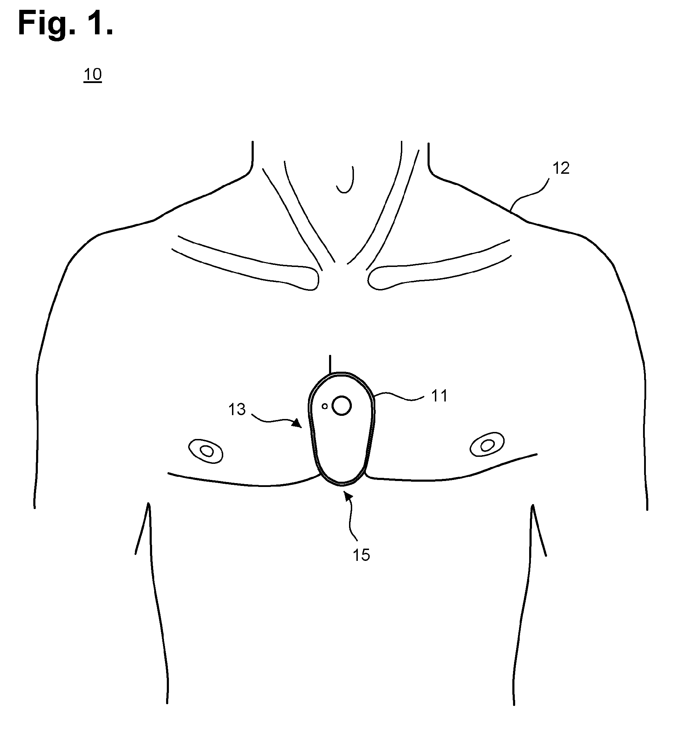

Primary care providers can be provided with a low-cost and highly-accessible ambulatory electrocardiographic monitor that generates, as appropriate, a referral to a medical specialist without mandating continuing primary care clinic oversight or active involvement. FIGURE 1 is a front anatomical diagram 10 showing placement of an ambulatory electrocardiographic (ECG) monitor 11 on a male patient 12. The monitor 11 is applied to the patient 12 on the skin's surface over the center of the sternum 15 with the sensing electrodes aligned along the midline of the patient's chest 13. Placement of the monitor 11 on female patients is encumbered by the presence of breasts and can require additional considerations to ensure safety, comfort, and long-term adhesion over the course of the monitoring period. On a female patient (not shown), the monitor 11 is placed between the breasts in the upper portion of the intermammary cleft, such as described in commonly-assigned U.S. Patent application, entitled "Ambulatory Electrocardiographic Monitor for Providing Ease of Use in Women and Method of Use," Serial No. 12/901,428, filed October 8, 2010, pending, the disclosure of which is incorporated by reference. The present discussion will primarily focus on placement of a monitor 11 on patients that that lack large-girthed, fatty, or well-developed breasts. For clarity, the term "male" will apply to individuals in this entire class of patients without regard to age, gender, or other physical characteristics or traits not germane to the selection of the monitoring site and placement of a monitor 11 on the patient's chest.

The monitor 11 may be applied in-clinic by a primary care provider, or by the patient herself, for instance, under a physician's orders after first obtaining the monitor 11 from a pharmacy or other authorized dispensary, such as described in commonly-assigned U.S. Patent application, entitled "Computer-Implemented System and Method for Mediating Patient-Initiated Physiological Monitoring," Serial No. 12/901,455, filed October 8, 2010, pending, the disclosure of which is incorporated by reference. The monitor 11 is typically used over a 24-48 hour period, but the monitoring period could be extended from seven days up to 30 days through use of a series of monitors. During monitoring, the patient 12 engages in activities of daily living, while the monitor 11 unobtrusively monitors and collects ECG data. Recording commences upon physical application of the monitor 11 and ends when the monitor 11 is removed, typically by the patient 12. Along with the monitor 11, the patient 12 receives instructions for having the monitor 11 processed post-monitoring, which can be performed by a monitoring, consultation, and specialist referral center, such as described in commonly-assigned U.S. Patent application, entitled "Computer-Implemented System And Method For Evaluating Ambulatory Electrocardiographic Monitoring of Cardiac Rhythm Disorders," cited *supra.* As appropriate, the patient 12 is referred to a medical specialist for follow up care, such as described in commonly-assigned U.S. Patent application, entitled "Computer-Implemented System and Method for Facilitating Patient Advocacy through Online Healthcare Provisioning," Serial No. 12/901,433, filed October 8, 2010, pending, the disclosure of which is incorporated by reference.

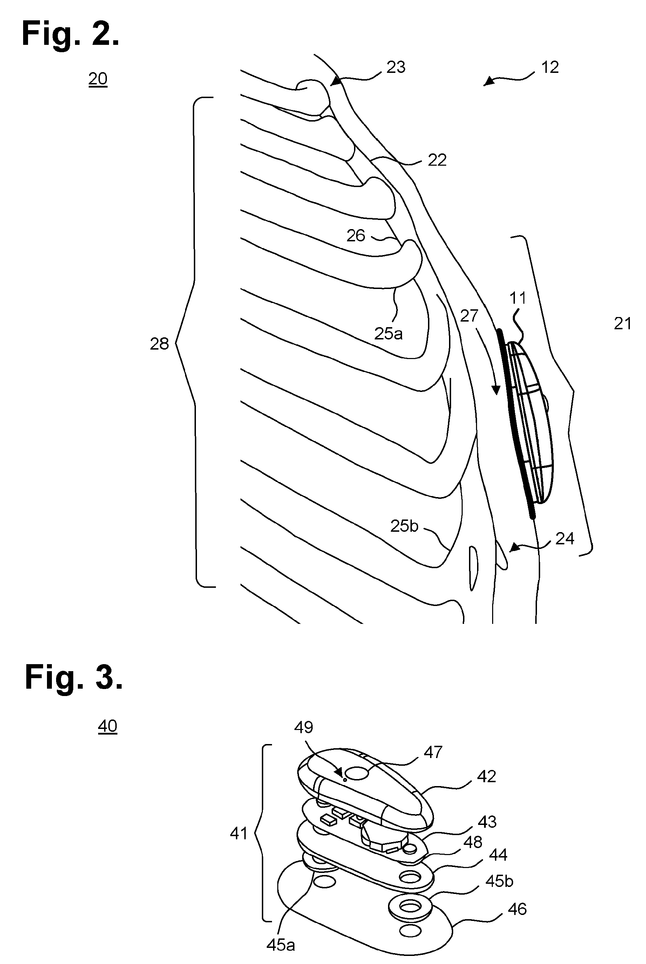

Proper placement of the monitor 11 is critical to recording high quality ECG data. FIGURE 2 is a cutaway anatomical diagram 20 showing placement of the ambulatory electrocardiographic monitor 11 of FIGURE 1. The ambulatory monitor 11 is removably adhered onto the skin on the patient's chest 21 at midline, covering the center third of the chest 21 over the sternum 26, roughly between the third and fifth ribs 25a, 25b and approximately centered between the suprasternal notch 23 on the superior border of the manubrium and the xiphoid process 24 on the inferior border of the sternum 26.

The midline sternum-centered monitoring site enables high P-wave and QRS-wave acquisition and provides several additional benefits over other more typical cutaneous monitoring locations, like those locations over the left upper chest or in the left inframammary crease. First, electrical current originating from the atria and ventricles flow directly underneath the sternum 26 providing excellent P waves and QRS waves necessary for cardiac rhythm diagnosis. Signal quality is further improved by minimizing the depth of tissue, and noise thus generated by moving tissue, between the monitor's electrodes and the heart. Tissue depth is fairly consistent at sternal midline where variations in the patient's weight and physical topology least interfere with ECG signal pickup. The midline sternum-centered location enables the monitor's electrodes to record an ECG of optimal signal quality from a location immediately above the strongest signal-generating aspects of the heart. Further, the surface of the skin located over the midline sternum-centered location remains relatively stationary, despite body motion or movement of underlying breasts, muscle, or other body tissue. Movement of the skin surfaces of the upper thoracic region can be of significant moment, particularly on obese patients or adult women with large breasts. Adhering the monitor 11 to a body position of minimal movement helps ensure that the monitor 11 remains adhered to the patient 12 throughout the entire monitoring period, as further described *infra.*

The ambulatory ECG monitor is constructed to provide low cost widespread use, with a particular emphasis in improving patient care at the primary care medical practice level. FIGURE 3 is an exploded perspective view 40 of an ambulatory electrocardiographic monitor 41 in accordance with one embodiment. Physically, when viewed from above, the monitor 41 has an elongated triangular shape with rounded vertices, such as described in commonly-assigned U.S. Design Patent No. D639,437, issued June 7, 2011; the disclosure of which is incorporated by reference, with dimensions of approximately 3.8cm (1.5in) wide and 7.6cm (3.0in) long with a pair of electrodes 48 spaced less than 6cm apart. The monitor 41 weighs about 14.2g (0.5oz) when assembled with electrodes 48 and a waterproof housing for the ECG recording circuitry, although a weight of up to 28g (1.0oz) would be acceptable. In one embodiment, the pair of electrodes 48 have an approximately 5.33cm spacing, although other electrode spacing, generally less than 6cm, and combinations of three or more electrodes could also be used. When adhered onto a patient's sternum, the narrowest part of the monitor 41 faces downwards towards the patient's feet. On a female patient, the narrow part fits partway into the upper intermammary cleft.

The monitor 41 is constructed in a modular fashion and includes a flexible housing and standoff-separated skin adhesion assembly. The housing includes a cover 42, printed circuit board (PCB) 43, and cover base 44, and the skin adhesion assembly includes a set of standoffs 45a, 45b, a layer of skin adhesive 46, and a set of electrodes 48. The housing protects the electronic components for sensing and recording ECG data, as further described below with reference to FIGURE 7, which are affixed to the PCB 43. The cover 42 conformably fits against the edges of the cover base 44. The cover 42 and cover base 44 form a water resistant enclosure that fully enclose the PCB 43. In a further embodiment, the housing 61 is vented, which allows the cover 42 to slightly "give" when pressed. A button 47 is formed on the top surface of the cover 42 that engages a switch on the PCB 43, which the patient can press during monitoring to mark an event occurrence, such as onset of dyspnea. An indicator light 49, such as a light emitting diode, visually signals the patient 12 that the monitor 11 is working. A steady light signifies normal operation, while a blinking light indicates a problem.

The outer materials are selected for extended term use. The cover 42 and cover base 44 are both constructed from flexible bio-safe materials, such as plastic, silicon, or foam, and can be vacuum-formed, extruded, or die cut. The adhesive layer 46 is constructed using an adhesive fabric or cloth, which can be woven, as well as latex, foam, and other materials that sufficiently resist the twisting and torquing of the skin's surface. In a further embodiment, triangular cutouts or "darts" are cut into the periphery of the adhesive layer 46 to more closely conform the adhesive layer 46 to an uneven or contoured skin surface. Other materials and methods of manufacture are possible.

The housing and skin adhesion assembly facilitate long term monitoring. Continuous and uninterrupted wear of the monitor 41 over the entire course of monitoring may be impracticable for every patient. Skin sensitivities, allergies, irritation, and similar factors have an effect on a patient's ability to tolerate the wearing of the monitor 41 for an extended period. Similarly, oil on the skin's surface, perspiration, and overall physical hygiene can affect monitor adhesion. As a result, the housing can be separated from the skin adhesion assembly to allow the patient 12 to reposition or replace the skin adhesion assembly. The set of electrodes 48 fit within set of standoffs 45a, 45b and a set of holes or "gel wells," in the skin adhesive layer 46. In turn, the skin adhesive layer 46 is affixed to the cover base 44 through a combination of a pair of snap-on or similar form of removable connectors facing downwardly from the PCB 44 and adhesive applied to the upward facing surfaces of the standoffs 45a, 45b.

To facilitate overall long term monitoring through a series of short term monitoring periods, the housing can be separated from the skin adhesion layer and either a new skin adhesion layer can be applied, or the existing skin adhesion layer can be repositioned. Either the same housing or a new housing can be used during successive periods of monitoring. When the same housing is reused, the recording circuitry compensates for disconnection and reconnection of the sensing electrodes by stopping recording of ECG data during the gap in monitoring, as sensed by disconnection from the set of electrodes 48. The recording circuitry thereafter resumes recording upon being reconnected to a set of electrodes 48. If necessary, the patient 12 may choose to take a break and allow her skin to "breathe" between applications of the skin adhesion layer.

In one embodiment, the monitoring circuit for ECG recording used by the monitor 10 operates under microprogrammed control on a single channel of analog input signals. The signals originate as cardiac action potentials sensed from the skin's surface by a single sensing electrode pair, although multiple sensing electrode pairs could be employed with modifications to the monitoring circuit to factor in multiple input signal channels. The analog input signals are converted into digitized form and encoded for efficient compressed data storage in non-volatile memory. The monitoring circuit injects a reference feedback signal into both the analog input signal path and the patient's body. Thus, noise generated by the electronics is integrated into the input signals, rather than being filtered or rejected. The monitoring circuit is thereby able to operate unshielded, with no filtering, and through minimal power filtering components, which thereby eliminates the need for either the cover 42 or cover base 44 to include physical noise shielding is eliminated through unique printed circuit board design and layout, as well as careful selection of electronic components that naturally dampen received noise. As well, the digitization and compression of the original low noise analog signal requires less memory to store long term ECG data.

Referring back to FIGURE 2, the body's surface over the sternum 26 is inherently uneven, even in children, due to the underlying bone structure of the body of the sternum 26 and ribs 28, as well as the muscle, fat, skin, and various tissue that cover the sternum 26 and adjacent regions. The front surface of the body of the sternum 26 is slightly convex in the east-west directions and the sternum's front surface angles in towards the thoracic cavity from around the fourth intercostal space 27 down to the xiphoid process 24 in the north-south directions. In the elderly, particularly in older males, the east-west convexity can become increasingly pronounced with age, resulting in a so-called "pigeon-chested" appearance.

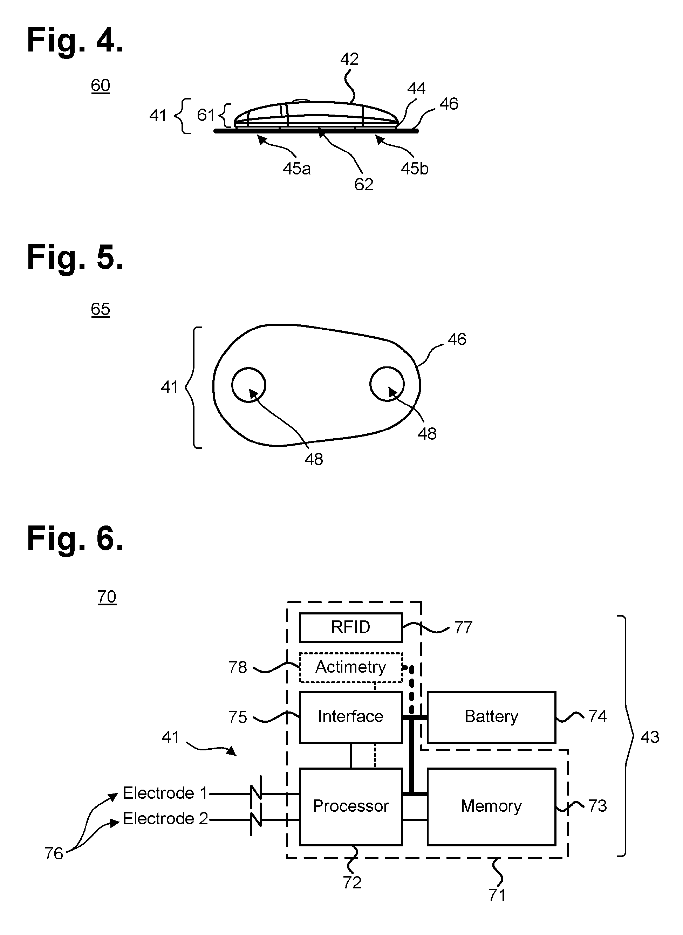

The sternal surface is non-planar, even in men, and the surface of the skin over the sternum has a subtle three-dimensional topography. A proper understanding of this topography is critical to device design. Conforming fit and secure adhesion to this inherently uneven surface are provided through two interconnected structures: a flexible housing and standoff-separated skin adhesion assembly. FIGURES 4 and 5 are respectively side and bottom views 60, 65 of the ambulatory electrocardiographic monitor 41 of FIGURE 3. The monitor 41 must adhere to the sternum 26 during the monitoring period. The cover 42 and cover base 44 provide a housing 61 for the monitor's electronic components. In one embodiment, the PCB 43 is about 0.02" thick, which allows the PCB 43 to conform to the east-west convexity of the sternum 26 and to the natural north-south inward curve towards the xiphoid process 24. The flexibility of the housing 61 is integral to the design to comfortably adhere to the sternal surface and ensure that ECGs can be recorded from the sternal location.

Objects adhered to the sternum 26 need to be able to both conform statically to the shape of the chest 21 and to accommodate dynamic torsional movement, as occurs during stretching, sleeping, and other body movement. The PCB 43 can bend axially and laterally, but the PCB's ability to stretch is limited by physical constraints on electronics packaging. To provide stretch, the monitor 41 utilizes a form of independent suspension that enables the skin adhesive layer 46 to stretch, as well as flex, independently of the housing 61. The monitor 41 is adhered to the patient's skin through a layer of skin adhesive 46 that is affixed to the bottom surface of the cover base 44 around the set of standoffs 45a, 45b. The skin adhesive layer 46 is slightly larger than the bottom of the cover base 44 by about 0.125in, although other shapes, sizes, and dimensions could be used, including shapes that differ significantly from the top profile of the cover base 44. The set of electrodes 48 are removably affixed to a pair of snap-on connectors facing downwardly from the PCB 44 and are electronically connected to the PCB's circuitry. Other types of connectors that allow the set of electrodes 48 to be removably affixed could also be used. The set of electrodes 44 fit within openings formed in the set of standoffs 45a, 45b and a set of holes 66a, 66b, or "gel wells," in the skin adhesive layer 46. The electrodes 44 are coated with a conductive gel that also assists with adhering the monitor 41 to the patient's chest 21. The independent suspension is provided through the set of two or more standoffs 46a, 46b that create a gap 62 of about 2.5mm (0.1in) between the bottom surface of the cover base 44 and the top surface of the skin adhesive layer 46. The heights of each of the standoffs 45a, 45b allow the monitor 41 to stay securely attached to the patient 12 during torsional movement, such as occurs when stretching or rolling over in bed. In one embodiment, the standoffs 45a, 45b have uniform heights of about 2.5mm (0.1in). In a further embodiment, the standoffs 45a, 45b can have non-uniform heights to help compensate for different chest surface contours. The gap 62 allows the housing 61 to "float" above the skin contact surface, while the skin adhesive layer 46 can flex and stretch along with the skin's surface on the patient's sternum chest 21. The single-point contact of each of the standoffs 45a, 45b thus allows the monitor 41 to accommodate the patient's twisting and turning movements and remain affixed without danger of peeling off.

The electronics package of each monitor facilitates low-cost extended wear use. FIGURE 6 is a functional block diagram 70 showing the groups of electronic components 71 of the ambulatory electrocardiographic monitor 41 of FIGURE 3. The monitor 41 is self-contained and operates under microprogrammed control, such as described in commonly-assigned U.S. Patent application, entitled "Microcontrolled Electrocardiographic Monitoring Circuit with Feedback Control," Serial No. 12/901,449, filed October 8, 2010, pending, and U.S. Patent application, entitled "Microcontrolled Electrocardiographic Monitoring Circuit with Differential Voltage Encoding," Serial No. 12/901,460, filed October 8, 2010, pending, the disclosures of which are incorporated by reference. Digitally-controlled ECG monitoring circuits provide the ability to handle the wide dynamic range occasioned by the short signal vector and low signal strength afforded by a midline sternum-centered ambulatory monitoring location.

In a functional sense, the electronic components 71 can be grouped into circuitry for a processor 72, memory 73, power supply or battery 74, data interface 75, and radio frequency identification (RFID) tag 77. The processor 72 is a discrete ECG recording circuit that operates under microprogrammed control on a single channel of analog input signals. To sense ECG data, the processor 72 interfaces to a set of external electrodes 76 through amplifiers and filters (not shown). Signals originate as action potentials sensed on the skin's surface by at least one of the electrodes 76 and a feedback signal is output through the other electrode 76. The sensed ECG data is processed into a stream of discrete digital values and encoded in the persistent non-volatile memory 73, which can be implemented as electrically-erasable programmable read-only memory (EEPROM) or "flash" memory. The data interface 75 enables the processor 71 to download recorded ECG data from the memory 73 and receive programming instructions. The processor 71, memory 72, and data interface 74 can be a single discrete integrated circuit or a set of individual components interconnected through data channels. The battery 74 is a conventional power cell or capacitor that provides power to the recording circuitry sufficient to enable extended operation.

In a further embodiment, either or both of the memory 73 and the battery 74 can be separately provided on the skin adhesion layer 46 to facilitate long term monitoring through use of a series of short term monitoring periods. Space for storing recorded ECG data and power for operating the recording circuitry are continually depleted. Providing the memory 73 and the battery 74 on the skin adhesion layer 46 enables those resources to be replenished, while enabling use of the same physical recording circuitry throughout the entire monitoring period.

The RFID tag 77 contains a unique identifier for the monitor that is either included on the PCB 43 with the other electronic components, or is embedded into the housing 61, such as within a foam-constructed cover 42. The RFID tag 77 is used during monitoring to pair a monitor 41 to a tracking number that can be used by the patient 12, referral center, and physician or staff to track the physical whereabouts of the monitor 41 and to determine the post-monitoring status of diagnosis and follow up care. The RFID tag 77 is self powered or can be powered through the battery 74. The RFID tag 77 is accessed using standard RFID transmitter and receiver units. Other components in addition to or in lieu of the electronic components 71 are possible, such as used to record additional types of patient physiometry or to provide further onboard capabilities.

In a further embodiment, the electronic components 71 also include an actimetry sensor 78 to measure gross motor activity undertaken by the patient, such as through walking, running, changing posture or sleep position, and other body motions. For instance, the actimetry sensor 78 may record movement, which indicates that the patient was climbing stairs at the same time that an increase in heart rate was recorded by the monitor 11. Particularly, when actigraphy is combined with the patient's subjective impressions as contemporaneously recorded in his diary, the physician can confirm or better understand hemodynamic changes and other aspects of cardiac physiology as reflected in the recorded ECG data.

The monitor 41 may be fully or partially disposable. For instance, the electronic components 71 on the PCB 43 may be refurbished and recycled for multiple uses, while the housing 61 and skin adhesive 46 would be disposed after a single use. During refurbishment, the battery 74 would be replaced and the memory 73 wiped clean. Alternatively, the entire monitor 41 may be used only once, followed by appropriate disposal.

While the invention has been particularly shown and described as referenced to the embodiments thereof, those skilled in the art will understand that the foregoing and other changes in form and detail may be made therein without departing from the spirit and scope.

## Claims

1. A method for performing ambulatory electrocardiographic (ECG) monitoring, comprising:
provisioning an ambulatory ECG monitor (11) comprising a plurality of sensing electrodes (48) coupled to self-powered sensing circuitry (71);
locating a monitoring site (15) on the surface of a patient's chest at midline and above the body of the sternum adjacent to the fourth and fifth intercostal spaces;
aligning and placing the electrodes (48) along the midline and removably adhering the ambulatory ECG monitor (11) to the monitoring site (15) for the duration of monitoring; and
sensing ECG data through the sensing electrodes (48) at the monitoring site (15) and recording the sensed ECG data into the sensing circuitry (71).

2. A method according to Claim 1, further comprising:
selecting a circuit board (43) exhibiting axial and lateral flexibility;
provisioning the sensing circuitry (71) on the selected circuit board (43) and enclosing the sensing circuitry (71) within a housing (61); and
conformably flexing the circuit board (43) within the housing (61) along the contours of the chest's surface.

3. A method according to Claim 1, further comprising:
enclosing the sensing circuitry (71) within a housing (61);
selecting a layer of skin adhesive (46) and a set of standoffs (45a, 45b);
attaching the skin adhesive layer (46) to a bottom surface of the housing (61) separated by the set of standoffs (45a, 45b); and
conformably adhering the skin adhesive layer (46) to the chest's surface with the housing (61) separated from the chest's surface by a gap formed by the heights of the set of standoffs (45a, 45b).

4. A method according to Claim 3, further comprising one of:
defining the skin adhesive layer (46) in a shape comparable to the shape of the bottom of the housing (61); and
defining the skin adhesive layer (46) in a shape differing from the shape of the bottom of the housing (61).

5. A method according to Claim 1, further comprising:
fashioning a housing (61) comprised of an elongated triangular shape with rounded vertices; and
enclosing the sensing circuitry (71) within a housing (61), wherein the housing (61) is removably adhered to the monitoring site (15) with the narrowest part of the triangular shape facing towards the patient's feet.

6. An ambulatory electrocardiographic (ECG) monitor (11) with conformal shape and independent suspension, comprising:
a flexible ECG circuitry body, comprising:
self-powered ECG sensing circuitry (71) comprising a processor, memory, and finite power supply;
a circuit board (43) exhibiting axial and lateral flexibility and upon which the sensing circuitry (71) is comprised; and
a housing (61) enclosing the circuit board (43);
a plurality of sensing electrodes (48) coupled to the processor, which processes and stores sensed ECG data into the memory; and
a skin adhesion assembly, comprising:
a layer of skin adhesive (46) facing a contact surface; and
a set of standoffs (45a, 45b) affixed to and defining a gap between the skin adhesive layer (46) and a bottom surface of the housing (61) of the circuitry body.

7. A monitor (11) according to Claim 6, further comprising:
a hole defined through a center of each of the standoffs (45a, 45b), wherein the sensing electrodes (48) are positioned in each of the holes facing the contact surface.

8. A monitor (11) according to Claim 6 or 7, wherein one or more cutouts are defined around the periphery of the skin adhesive.

9. A monitor (11) according to any one of Claims 6 to 8, further comprising:
a radio frequency identification tag comprised with the flexible ECG circuitry body and providing a unique identifier.

10. A monitor (11) according to any one of Claims 6 to 9, further comprising:
an actimetry sensor coupled with the sensing circuitry (71) and storing gross motor activity data into the memory.

11. A monitor (11) according to any one of Claims 6 to 10, wherein at least one of the memory and the finite power supply are comprised on the skin adhesive layer (46) instead of the circuitry body.

12. A monitor (11) according to any one of Claims 6 to 11, wherein the skin adhesive layer (46) comprises adhesive fabric, cloth, foam, and latex.

13. A monitor (11) according to any one of Claims 6 to 12, wherein the skin adhesive layer (46) is defined in a shape comparable to one of the shape of the bottom of the housing (61) and a shape differing from the shape of the bottom of the housing (61).

14. A monitor (11) according to any one of Claims 6 to 13, wherein the plurality of electrodes (48) are spaced less than 6 cm apart.

15. A monitor (11) according to any one of Claims 6 to 14, wherein the monitor (11) weighs not more than 28g (1.0oz).
